# EUROPÄISCHE PATENTSCHRIFT

(11) **EP 0 591 821 B1**
(45) Veröffentlichungstag und Bekanntmachung des Hinweises auf die Patenterteilung: **15.01.1997**
(21) Anmeldenummer: 93115672.3
(22) Anmeldetag: 29.09.1993
(51) Int. Cl.: D06M 15/263, D06M 15/03, C08F 251/00, A61L 15/62, A61L 15/28

(54) **Zur Kompostierung geeigneter Vliesstoff, gebunden mit einem Saccharidpfropfpolymerisat**
Fleece material suitable for composting, bonded with a saccharid graft polymer
Voile textile qui convient pour le compost, lié avec un polymère greffé de saccharide

(30) Priorität: 06.10.1992 DE 4233610
(43) Veröffentlichungstag der Anmeldung: 13.04.1994
(73) Patentinhaber: BASF Aktiengesellschaft, 67063 Ludwigshafen (DE)
(72) Erfinder: Wuestefeld, Renate, Dr., D-67105 Schifferstadt (DE); Schumacher, Karl-Heinz, Dr., D-67435 Neustadt (DE); Kirsch, Howard Peter, Dr., D-67251 Freinsheim (DE)

(56) Entgegenhaltungen:
- EP-A- 0 019 169
- EP-A- 0 134 449
- EP-A- 0 334 515
- EP-A- 0 405 921
- EP-A- 0 408 099
- EP-A- 0 499 774
- EP-A- 0 536 597
- FR-A- 2 305 452

## Beschreibung

Die Erfindung betrifft einen gebundenen Vliesstoff, enthaltend als Binder 5 bis 100 Gew.-%, bezogen auf das Gewicht der eingesetzten biologisch abbaubaren Fasern, eines Polymerisates, das eine Glastemperatur von -70 bis +40°C aufweist und das herstellbar ist durch radikalische Polymerisation von ethylenisch ungesättigten Monomeren in wäßrigem Medium in Gegenwart eines Saccharids mit einem gewichtsmittleren Molekulargewicht von 1000 bis 25000, wobei das Monomerengemisch 0,5 bis 15 Gew.% N-Alkylolamide von 3 bis 10 C-Atome aufweisenden α,β-monoethylenisch ungesättigten Carbonsäuren, Acrylamidoglykolsäure, Methacrylamidoglykolsäure und/oder deren Ether, Ester oder Etherester mit alkoholen mit bis zu 12 C-Atomen, enthält.

Als Vliesstoffe werden textile Flächengebilde, die im allgemeinen flexibel sind, zusammengefaßt, die durch Verfestigen lockerer Anhäufungen von Einzelfasern, sog. Faservliesen, hergestellt werden. Die Verfestigung oder das Binden von Faservliesen durch Imprägnieren mit waßrigen Polymerisatdispersionen und anschließendes Abdampfen des Wassers ist allgemein bekannt.

Vliesstoffe haben aufgrund ihrer im Vergleich zu Geweben und Gewirken verhältnismäßig geringen Herstellungskosten eine weite Anwendung in textilen Wegwerfprodukten gefunden. Beispiele hierfür sind Windeln, Putz- und Wischtücher oder medizinische Artikel wie Monatsbinden oder Tücher und Decken.

Diese Produkte erfordern allgemein eine hohe Festigkeit sowohl im trockenen als auch im nassen Zustand in Kombination mit einem weichen Charakter. Daneben werden für Produkte, die mehr oder weniger innigen Kontakt zum Menschen haben, steigende Anforderungen an deren toxikologische Unbedenklichkeit gestellt.

Mit der mengenmäßig immer stärker auftretenden Verbreitung dieser Wegwerfartikel und der zunehmenden gesellschaftlichen Diskussion der Müllproblematik tritt die Frage nach der Entsorgung der gebrauchten Artikel in den Vordergrund. Die Entsorgung erfolgt bisher vor allem durch Ablagerung in Mülldeponien. Angesichts knapp werdender Deponieräume und abnehmender gesellschaftlicher Akzeptanz dieser Entsorgungstechnik ist eine Entsorgung der vliesstoffhaltigen Wegwerfartikel durch Kompostierung als ideale Alternative zu den bisherigen Entsorgungstechniken höchst wünschenswert. Die Produkte sollten daher gut kompostierbar sein. Darunter versteht man im allgemeinen den erfolgreichen Verrottungsprozeß in Kompostieranlagen. Die Produkte sollen daher in Kontakt mit Erde schnell ihre Festigkeit verlieren und zerfallen.

In DE-A-23 61 468 wird ein gebundener Vliesstoff beschrieben, der aus kurzen Fasern wie Cellulosefasern und einem Bindemittel besteht, welches als Abmischung aus einem Polymerisat aus ethylenisch ungesättigten Monomeren und natürlich vorkommenden Polymeren wie Gelatine, Collagen oder Stärke besteht. Die Mischung wird aus waßrigem Medium auf den Vliesstoff aufgebracht und das Wasser entfernt. Die so gebundenen Vliesstoffe befriedigen jedoch nicht, da sie ungünstige Eigenschaften aufweisen. So ist die Naßfestigkeit und die Weichheit dieser Produkte ungünstig.

US-A-3 651 210 beschreibt ein reaktives Copolymer aus bestimmten, u.a. oxiranhaltigen Monomeren und Proteinen wie Casein oder Soja als Beschichtungsmaterial oder Klebstoff. Aus der DE-A-41 08 170 war es bekannt, ethylenisch ungesättigte Monomere in Gegenwart von Proteinen wie Casein radikalisch in Emulsion zu polymerisieren. Aus den Produkten werden Folien und Beschichtungen, beispielsweise auf Papier, hergestellt. Beide Schriften geben keinerlei Anregung, in Gegenwart von Stärke hergestellte Polymerisate zum Binden von Faservliesen einzusetzen. Darüber hinaus werden zu wenig wasserfeste und zu steife Vliesstoffe erhalten, wenn man die in DE-A-41 08 170 offenbarten Pfropfpolymerisate zum Binden einsetzt.

Die EP-A-345 566 betrifft Vliesbinder auf Basis eines Copolymers aus hydroxylgruppenfreien Monomeren und hydroxylgruppenhaltigen Komponenten wie Polyvinylalkohol, Stärke, Stärkederivaten oder kolloidaler Cellulose. Diese Vliesbinder werden vernetzt durch Zusatz von 1,3-Dimethyl-4,5-dihydroxiimidazolidinon zum Polymeren. Diese Produkte weisen jedoch eine schlechte Naßfestigkeit und eine ungünstige Weichheit auf.

Die EP-A-0 019 169 offenbart einen gebundenen Vliesstoff, enthaltend als Binder ein Polymerisat, das herstellbar ist durch radikalische Polymerisation eines Gemisches aus ethylenisch ungesättigten Monomeren enthaltend N-Alkylolamide von 3-10 C-Atomen aufweisenden alpha, beta-ungesättigten Carbonsäuren und gegebenenfalls Acrylamidoglykolsäure bzw. Methacrylamidoglykolsäure.

Aufgabe der vorliegenden Erfindung war es daher, die Nachteile des Standes der Technik zu vermeiden und gebundene Vliesstoffe mit ausgewogenen Eigenschaften bereitzustellen, die kompostierbar sind. Die Produkte sollten bei einer hohen Trockenfestigkeit, einer guten Naßfestigkeit und einem weichen Charakter eine gute Kompostierbarkeit aufweisen.

Demgemäß wurden die in Anspruch 1definierten Vliesstoffe gefunden. Weiterhin wurde ein Verfahren zum Verfestigen von Faservliesen gefunden. Bevorzugte Ausführungsformen sind den Unteransprüchen zu entnehmen.

Als Material für die Faservliese werden Fasern, eingesetzt, die biologisch abbaubar sind. Es handelt sich im allgemeinen um Fasern mit einem Durchmesser von 0,002 bis 0,1 mm, bevorzugt 0,01 bis 0,05 mm, der beispielsweise in üblicher Weise mit Hilfe von elektronmikroskopischen Aufnahmen bestimmt werden kann. Im allgemeinen werden natürliche Fasern cellulosischen Ursprungs, wie Viskosefasern oder Zellstoffasern oder synthetische Fasern, wie aliphatische Polyesterfasern, beispielsweise auf Basis von Copolymerisaten aus 3-Hydroxybutyrat, 3-Hydroxyvalerat und 4-Hydroxyvalerat eingesetzt, wie sie in EP-A-466 050 beschrieben sind.

Die Formung von Faservliesen aus den Fasern ist allgemein bekannt (Römpp, Chemielexikon, Georg Thieme Verlag, Stuttgart - New York, 9. Auflage, Seite 4550). Es kann sich um Wirrfaservliese oder bevorzugt gerichtete Faservliese ohne oder mit mechanischer Vorverfestigung, beispielsweise durch Nadeln, Verwirbeln oder Nähwirken, handeln.

Zum Binden der Faservliese werden 5 oder mehr, bevorzugt 11 oder mehr, insbesondere 15 oder mehr, speziell 20 oder mehr Gew.-% und 100 oder weniger, bevorzugt 50 oder weniger, insbesondere 35 oder weniger Gew.-%, jeweils bezogen auf die eingesetzte Menge an Faservlies, des Polymerisates verwendet.

Die Pfropfung von ethylenisch ungesättigten Monomeren auf Stärke ist bekannt, beispielsweise aus EP-A-134 449, EP-A-334 515, EP-A-408 099 und DE-A-41 33 193. Stärkepfropfpolymerisate werden empfohlen für Papierleimung (EP-A-257 412), als Zusatz zu Wasch- und Reinigungsmitteln oder zu Papierstreichmassen (EP-A-441 197), für Formen für den Metallguß (DE-A-41 33 190.7) und für Schleifmittel (DE-A-41 33 191.5).

Die erfindungsgemäß eingesetzten Polymerisate weisen eine Glasübergangstemperatur von -70 bis +40, bevorzugt -60 bis 0°C, auf. Diese Glasübergangstemperatur ist in bekannter Weise nach ASTM 3418/82 (sog. "midpoint-Temperatur") meßbar.

Als radikalisch polymerisierbare Monomere kommen insbesondere monoethylenisch ungesättigte Monomere wie Olefine mit bis zu 4 C-Atomen, z.B. Ethylen, vinylaromatische Monomere mit bis zu 10 C-Atomen wie Styrol, α-Methylstyrol, ortho-Chlorstyrol oder Vinyltoluole, Vinyl- und Vinylidenhalogenide, wie Vinyl- und Vinylidenchlorid, Ester aus Vinylalkohol und 1 bis 18 C-Atome aufweisenden Monocarbonsäuren wie Vinylacetat, Vinylpropionat, Vinyl-n-butyrat, Vinyllaurat und Vinylstearat, Ester aus vorzugsweise 3 bis 6 C-Atome aufweisenden α,β-monoethylenisch ungesättigten Mono- und Dicarbonsäuren, wie insbesondere Acrylsäure, Methacrylsäure, Maleinsäure, Fumarsäure und Itaconsäure mit im allgemeinen 1 bis 12, vorzugsweise 1 bis 8 und insbesondere 1 bis 4 C-Atome aufweisenden Alkanolen, wie besonders Acrylsäure- und Methacrylsäuremethyl-, -ethyl-, -n-buytl-, -isobutyl- und -2-ethylhexylester, Maleinsäuredimethylester oder Maleinsäure-n-butylester, Nitrile der genannten α,β-monoethylenisch ungesättigten Carbonsäuren, wie Acrylnitril oder Methacrylnitril sowie C₄ bis C₈-konjugierte Diene, wie 1,3-Butadien und Isopren, in Betracht. In vielen Fällen hat es sich als günstig erwiesen, wenn der Anteil der Nitrile kleiner als 15 Gew.-%, bevorzugt 0 Gew.-%, der Gesamtmenge der eingesetzten Monomeren ist.

Die genannten Monomeren sind im wesentlichen in wäßrigem Medium nicht löslich und bilden in der Regel die Hauptmonomeren, die, bezogen auf die Gesamtmenge der eingesetzten Monomeren, normalerweise einen Anteil von mehr als 50 Gew.-% auf sich vereinen.

Es werden vernetzend wirkende Monomere in Mengen von 0,5 bis 15, bevorzugt von 0,5 bis 10 Gew.-%, insbesondere 1 bis 6 Gew.-%, bezogen auf die Gesamtmenge der verwendeten Monomeren, eingesetzt, die erst während der Trocknung des Polymerisates die Vernetzungsreaktion eingehen, wobei die Vernetzungsreaktion durch Tempern und ggfs. durch Zusatz von Katalysatoren, wie z.B. protonenabspaltende Substanzen wie Maleinsäure, Diammoniumhydrogenphosphat oder Ammoniumnitrat, forciert werden kann. Beispiele für solche Monomere sind N-Alkylolamide von 3 bis 10 C-Atome aufweisenden, α,β-monoethylenisch ungesättigten Carbonsäuren, unter denen das N-Methylolacrylamid und N-Methylolmethacrylamid bevorzugt sind. Besonders bevorzugt als vernetzend wirkendes Monomeres ist die Acrylamidoglykolsäure und Methacrylamidoglykolsäure und deren Ether, Ester oder Etherester mit Alkoholen wie Alkanolen mit bis zu 12 C-Atomen, beispielsweise Acrylamidomethoxyessigsäure, Acrylamidohydroxyessigsäuremethylester, Acrylamidomethoxyessigsäuremethylester, Methacrylamidomethoxyessigsäure, Methacrylamidohydroxyessigsäuremethylester, Methacrylamidomethoxyessigsäuremethylester, die entsprechenden Butyl- und -butoxyderivate, Acrylamidobutoxyessigsäurebutylester und Methacrylamidobutoxyessigsäurebutylester. Die Ammoniumsalze der genannten (Meth)acrylamidosäuren sind daneben auch geeignet. Diese Vernetzungssysteme spalten bei der Vernetzungsreaktion keinen toxikologisch bedenklichen Formaldehyd ab. Die freie (Meth)acrylamidoglykolsäure wird ganz besonders bevorzugt.

Von den genannten Monomeren verschiedene Monomere, die für sich polymerisiert üblicherweise wasserlösliche Homopolymerisate ergeben, werden im Normalfall lediglich als modifizierende Monomeren in Mengen, bezogen auf die Gesamtmenge der eingesetzten Monomeren, von weniger als 50 Gew.-%, in der Regel 0 bis 15, vorzugsweise 1 bis 10 Gew.-%, mit einpolymerisiert. Beispiele für derartige Monomere sind 3 bis 6 C-Atome aufweisende α,β-monoethylenisch ungesättigte Mono- und Dicarbonsäuren und deren Amide, wie z.B. Acrylsäure, Methacrylsäure, Maleinsäure, Fumarsäure, Itaconsäure, Acrylamid und Methacrylamid, ferner Vinylsulfonsäuren und deren wasserlösliche Salze, Vinylphosphonsäure, deren Salze und Ester mit Alkoholen mit bis zu 4 C-Atomen sowie n-Vinylpyrrolidon und 2-Hydroxyethlyacrylat, 3-Hydroxypropylacrylat oder Dimethylaminoethylacrylat.

Bevorzugte Polymerisate entstehen aus
- 70 bis 99,5 Gew.-% aus Estern der Acryl- und/oder Methacrylsäure mit 1 bis 12 C-Atome aufweisenden Alkanolen, Styrol, Butadien, Vinylacetat und/oder Vinylpropionat,
- 0,5 bis 15 Gew.-% vernetzender Monomerer und
- 0 bis 15 Gew.-% weiterer Monomerer.

Besonders bevorzugte Polymerisate entstehen aus Monomerengemischen aus 90 bis 99,5, bevorzugt 94 bis 99 Gew.-% Estern der Acryl und/oder Methacrylsäure mit 1 bis 12, insbesondere 1 bis 8 C-Atome aufweisenden Alkanolen und zu 0,5 bis 10, bevorzugt 1 bis 6 Gew.-% aus Acrylsäure, Methacrylsäure und/oder insbesondere Acrylamidoglykolsäure, Methacrylamidoglykolsäure und den oben genannten Derivaten dieser Säuren, wobei die Acrylamidoglykolsäure und die Methacrylamidoglykolsäure ganz besonders bevorzugt sind.

Die genannten Gew.-% beziehen sich jeweils auf die Gesamtmenge der eingesetzten ethylenisch ungesättigten Monomeren.

Die Polymerisation der genannten Monomeren erfolgt im allgemeinen nach dem Verfahren der radikalischen wäßrigen Emulsionspolymerisation. Es handelt sich um eine Pfropfpolymerisation in Anwesenheit von Sacchariden. Darunter werden im Sinne dieser Erfindung Monosaccharide, Oligosaccharide und Polysaccharide und ihre Derivate verstanden.

Zu den Monosacchariden zählen die Pentosen (C₅H₁₀O₅) und Hexosen (C₆H₁₂O₆). Von Oligosacchariden spricht man, wenn Monosaccharide zu beispielsweise Dimeren oder Trimeren verbunden sind. Bevorzugt werden Derivate der Mono- und Oligosaccharide eingesetzt wie ihre Acetale mit C₆- bis C₁₈-Alkanolen. Genannt seien Octyl-D-glucosid, Decyl-D-glucosid und Dodecyl-D-glucosid, Tetradecyl-D-glucosid, Hexadecyl-D-glucosid, Octadecyl-D-glucosid und deren Mischungen.

Die eingesetzten Polysaccharide sind mit Vorteil α-glycosidisch verknüpft.

Die eingesetzten Polysaccharide weisen ein gewichtsmittleres Molekulargewicht von 1000 bis 25000, bevorzugt von 1000 bis 13000, insbesondere von 2000 bis 9000 auf, meßbar durch Gelpermeationschromatographie in üblicher Art.

Die Polysaccharide können pflanzlichen oder tierischen Ursprungs, in Wasser löslich oder nur darin dispergierbar sein. Geeignet sind u.a. die sogenannten Quellstärken, die beispielsweise durch hydrothermische Behandlung von nativer Stärke erhältlich sind. Ferner eignen sich dünnkochende Stärken. Es handelt es dabei um mit Säuren oder Enzymen geringfügig abgebaute oder mit milden Oxidationsmitteln oxidierte Stärken, die auch in höheren Konzentrationen beim Kochen mit Wasser keine viskosen Kleister, sondern relativ dünne Flüssigkeiten ergeben. Außerdem sind säuremodifizierte Stärken geeignet, die durch Erwärmen einer wäßrigen Stärkesuspension unterhalb der Verkleisterungstemperatur in Gegenwart geringer Säuremengen gewonnen werden. Weiterhin kommen oxidativ modifizierte Stärken in Betracht. Als Oxidationsmittel können z.B. Chromsäure, Permanganat, Wasserstoffperoxid, Stickstoffdioxid, Hypochlorit oder Perjodsäure herangezogen werden. Als Ausgangsstärken sind prinzipiell alle nativen Stärken wie Getreidestärken (z.B. Mais, Weizen, Reis oder Hirse), Knollen- und Wurzelstärken (z.B. Kartoffeln, Tapiokawurzeln oder Arrowroot) oder Sagostärken geeignet. Vorteilhaft ist die Verwendung von Röstdextrinen, wie sie z.B. in der EP-A-408 099 sowie in der EP-A-334 515 beschrieben sind. Sie sind durch Erhitzen von feuchttrockner Stärke, meist in Anwesenheit geringer Mengen Säure, erhältlich. Typische Röstdextrine sind z.B. im Handel erhältliche Weiß- und Gelbdextrine; ferner zählen dazu Dextrine, die unter dem Warenzeichen Noredux® und Tackidex® vertrieben werden. Der Begriff Dextrin wird hier ganz generell für Stärkeabbauprodukte verwendet.

Mit ganz besonderem Vorteil wird jedoch die radikalische Emulsionspolymerisation in Gegenwart von verzuckerten Stärken durchgeführt. Hierbei handelt es sich um ein durch Hydrolyse in wäßriger Phase erhältliches Stärkeabbauprodukt. Hierbei werden wäßrige Polymerdispersionen erhalten, die neben hoher mechanischer und thermischer Stabilität auch gute rheologische Eigenschaften auch nach Lagerung aufweisen. Detailliertere Angaben zur Herstellung der genannten Stärken und Stärkederivate findet man in G. Tegge, Stärke und Stärkederivate, Behr's Verlag, Hamburg 1984. Selbstverständlich können die genannten Stärken und Stärkederivate in z.B. durch Veretherung oder Veresterung chemisch modifizierter Form erfindungsgemäß angewendet werden.

Diese chemische Modifizierung kann bereits an der Ausgangsstärke vor deren Abbau oder danach durchgeführt werden. Veresterungen sind sowohl mit anorganischen als auch mit organischen Säuren, deren Anhydriden oder Chloriden möglich. Von besonderem Interesse sind phosphatierte und acetylierte Derivate. Die gängigste Methode zur Veretherung ist die Behandlung mit organischen Halogenverbindungen, Epoxiden oder Sulfaten in wäßriger alkalischer Lösung. Besonders geeignete Ether sind Alkylether, Hydroxyalkylether, Carboxyalkylether und Allylether. Ferner kommen cyanalkylierte Derivate sowie Umsetzungsprodukte mit 2,3-Epoxipropyltrimethylammoniumchlorid in Betracht. Chemisch nicht modifizierte Produkte sind jedoch bevorzugt. Geeignet sind auch Abbauprodukte der Cellulose, beispielsweise Cellobiose und ihre Oligomeren.

Die erfindungsgemäß ganz besonders bevorzugt anzuwendenden verzuckerten Stärken sind als solche im Handel erhältlich (z.B. die C∗ PUR Produkte 01906, 01908, 01910, 01912, 01915, 01921, 01924, 01932 oder 01934 der Fa. Cerestar). Derartige verzuckerte Stärken sind von den Röstdextrinen u.a. dadurch chemisch verschieden, daß bei einem hydrolytischen Abbau in wäßrigem Medium (üblicherweise Suspensionen oder Lösungen), der in der Regel bei Feststoffgehalten von 10 bis 30 Gew.% sowie vorzugsweise säure- oder enzymkatalysiert vorgenommen wird, die Möglichkeit der Rekombination und Verzweigung im wesentlichen nicht gegeben ist, was sich nicht zuletzt auch in anderen Molekulargewichtsverteilungen äußert. So haben sich verzuckerte Stärken, die eine bimodale Molekulargewichtsverteilung aufweisen, erfindungsgemäß als besonders vorteilhaft erwiesen. Die Herstellung verzuckerter Stärken ist allgemein bekannt und u.a. in G. Tegge, Stärke und Stärkederivate, Behr's Verlag, Hamburg 1984, S. 173 und S. 220 ff., sowie in der EP-A-441 197 beschrieben.

Die verzuckerten Stärken sind normalerweise bei Raumtemperatur in Wasser vollständig löslich, wobei die Löslichkeitsgrenze in der Regel oberhalb von 50 Gew.-% liegt, was sich für die Herstellung der wäßrigen Polymerisatdispersionen als besonders vorteilhaft erweist.

Es hat sich ferner als günstig erwiesen, wenn die verzuckerten Stärken eine Unheitlichkeit U (definiert als Verhältnis von gewichtsmittlerem Molekulargewicht M_{w} zu zahlenmittlerem Molekulargewicht Mₙ (meßbar über Gelpermeationschromatographie); U charakterisiert die Molekulargewichtsverteilung) im Bereich von 6 bis 12 aufweisen.

Ferner ist es von Vorteil, wenn der Gewichtsanteil der verzuckerten Stärken, der ein Molekulargewicht unterhalb von 1000 aufweist, wenigstens 10 Gew.-%, jedoch nicht mehr als 70 Gew.-% beträgt.

Darüber hinaus ist es empfehlenswert, solche verzuckerten Stärken anzuwenden, deren Dextroseäquivalent DE 5 bis 40 beträgt. Der DE-Wert charakterisiert das Reduktionsvermögen bezogen auf das Reduktionsvermögen von wasserfreier Dextrose und wird nach DIN 10 308 Ausgabe 5.71, des Normenausschusses Lebensmittel und landwirtschaftliche Produkte, bestimmt (vgl. auch Günther Tegge, Stärke und Stärkederivate, Behr's Verlag, Hamburg 1984, S. 305).

Außerdem hat es sich gezeigt, daß in ihrem Eigenschaftsprofil besonders günstige wäßrige Polymerisatdispersionen dann erhalten werden, wenn man verzuckerte Stärken einsetzt, deren 40 gew.-%ige wäßrige Lösungen bei 25°C und einem Schergefälle von 75 s⁻¹ eine nach DIN 53 019 bestimmte dynamische Viskosität η⁴⁰ [Pa·s] von 0,005 bis 0,06, vorzugsweise von 0,005 bis 0,03, aufweisen.

Die während der radikalischen wäßrigen Emulsionspolymerisation anwesenden Saccharide können sowohl als einzige Dispergiermittel als auch im Gemisch mit anderen grenzflächenaktiven Substanzen anwesend sein. Sie sind in den wäßrigen Polymerisatdispersionen normalerweise in Mengen von, bezogen auf die Menge an eingesetzten Monomeren, 1 bis 50, bevorzugt 3 bis 30, Gew.-% enthalten.

Als begleitende grenzflächenaktive Substanzen kommen prinzipiell die ansonsten als Dispergiermittel üblicherweise eingesetzten Schutzkolloide und Emulgatoren anionischer, kationischer oder nichtionischer Art in Betracht. Eine ausführliche Beschreibung geeigneter Schutzkolloide findet sich in Hoüben-Weyl, Methoden der organischen Chemie, Band XIV/1, Makromolekulare Stoffe, Georg-Thieme-Verlag, Stuttgart, 1961, S. 411 bis 420. Vorzugsweise werden als begleitende grenzflächenaktive Substanzen ausschließlich Emulgatoren eingesetzt, deren Molekulargewichte im Unterschied zu den Schutzkolloiden üblicherweise unter 2000 liegen. Selbstverständlich müssen im Falle der Verwendung von Gemischen grenzflächenaktiver Substanzen die Einzelkomponenten miteinander verträglich sein, was im Zweifelsfall an Hand weniger Vorversuche überprüft werden kann. Vorzugsweise werden anionische und nichtionische Emulgatoren als begleitende grenzflächenaktive Substanzen verwendet. Gebräuchliche begleitende Emulgatoren sind z.B. ethoxylierte Fettalkohole (Ethoxylierungsgrad (EO): 3 bis 50, Alkylrest: C₈ bis C₃₆), ethoxylierte Mono-, Di- und Tri-Alkylphenole (EO-Grad: 3 bis 50, Alkylrest: C₄ bis C₉), ethoxylierte Alkanole (EO-Grad: 4 bis 30, Alkylrest: C₁₂ bis C₁₈), ethoxylierte Alkylphenole (EO-Grad: 3 bis 50, Alkylrest: C₄ bis C₉), Alkalimetallsalze von Dialkylestern der Sulfobernsteinsäure sowie Alkali- und Ammoniumsalze von Alkylsulfaten (Alkylrest: C₈ bis C₁₂), von Alkylsulfonsäuren (Alkylrest: C₁₂ bis C₁₈) von Alkyldiphenyloxidsulfonsäuren (Alkylrest: C₁₂ bis C₁₈) und von Alkylarylsulfonsäuren (Alkylrest: C₉ bis C₁₈) und die entsprechenden Säuren. Weitere geeignete Emulgatoren finden sich in Hoüben-Weyl, Methoden der organischen Chemie, Band XIV/1, Makromolekulare Stoffe, Georg Thieme Verlag, Stuttgart, 1961, Seiten 192 bis 208. Bevorzugt sind solche grenzflächenaktiven Substanzen, die sich biologisch abbauen lassen und die selbst oder deren Abbau- und Folgeprodukte nicht toxikologisch bedenklich sind wie Natriumlaurylsulfat. Die grenzflächenaktiven Substanzen werden in der Regel in Mengen von O bis 5, bevorzugt 0,1 bis 1 Gew.-%, bezogen auf die Menge der zu polymerisierenden Monomeren, mitverwendet.

Die Polymerisationstemperatur beträgt in der Regel 30 bis 95, vorzugsweise 75 bis 90°C. Das Polymerisationsmedium kann sowohl nur aus Wasser, als auch aus Mischungen aus Wasser und damit mischbaren Flüssigkeiten wie Methanol bestehen. Vorzugsweise wird nur Wasser verwendet. Die Emulsionspolymerisation wird in üblichen Vorrichtungen durchgeführt, die mit Mischorganen versehen sind, z.B. mit Rührern ausgestatteten Kolben, Kesseln, Autoklaven und zylinderförmigen Reaktoren. Sie kann diskontinuierlich oder kontinuierlich, z.B. in Kesselkaskaden oder in anderen miteinander verbundenen Polymerisationsvorrichtungen durchgeführt werden. Sie kann sowohl als Batchprozeß als auch in Form eines Zulaufverfahrens, einschließlich Stufen- oder Gradientenfahrweise, durchgeführt werden. Bevorzugt ist das Zulaufverfahren, bei dem man einen Teil des Polymerisationsansatzes vorlegt, auf die Polymerisationstemperatur erhitzt, anpolymerisiert und anschließend den Rest des Polymerisationsansatzes, üblicherweise über mehrere räumlich getrennte Zuläufe, von denen einer oder mehrere die Monomeren in reiner oder in emulgierter Form enthalten, kontinuierlich, stufenweise oder unter Überlagerung eines Konzentrationsgefälles unter Aufrechterhaltung der Polymerisation der Polymerisationszone zuführt. In anwendungstechnisch vorteilhafter Weise enthält die Vorlage und/oder der Monomerenzulauf geringe Mengen an Emulgatoren, bezogen auf die Gesamtmenge der zu polymerisierenden Monomeren in der Regel weniger als 0,5 Gew.-%, um die Oberflächenspannung des Dispergiermediums zu reduzieren und so das Einrühren zu erleichtern. Häufig werden die Monomeren daher in mit diesen Hilfsemulgatoren voremulgierter Form der Polymerisationszone zugeführt. Mit Vorteil ist die Gesamtmenge des zu verwendenden Saccharids in der wäßrigen Vorlage im allgemeinen in gelöster Form enthalten.

Als radikalische Polymerisationsinitiatoren kommen alle diejenigen in Betracht, die in der Lage sind, eine radikalische wäßrige Emulsionspolymerisation auszulösen. Es kann sich dabei sowohl um Peroxide, z.B. Alkalimetallperoxidisulfate, Ammoniumperoxidisulfat oder H₂O₂, als auch um Azoverbindungen handeln.

Geeignet sind auch kombinierte Systeme, die aus wenigstens einem organischen Reduktionsmittel und wenistens einem Peroxid und/oder Hydroperoxid zusammengesetzt sind, z.B. tert.-Butylhydroperoxid und das Natriummetallsalz der Hydroxymethansulfinsäure oder Wasserstoffperoxid und Ascorbinsaure. Ferner eignen sich kombinierte Systeme, die darüber hinaus eine geringe Menge einer im Polymerisationsmedium löslichen Metallverbindung, deren metallische Komponente in mehreren Wertigkeitsstufen auftreten kann, enthalten, z.B. Ascorbinsäure/Eisen(II)sulfat/Wasserstoffperoxid, wobei anstelle von Ascorbinsäure auch häufig das Natriummetallsalz der Hydroxymethansulfinsäure, Natriumsulfit, Natriumhydrogensulfit oder Natriummetallbisulfit und anstelle von Wasserstoffperoxid tert.-Butylhydroperoxid oder Alkalimetallperoxidisulfate und/oder Ammoniumperoxidisulfate angewendet werden. In der Regel beträgt die Menge der eingesetzten radikalischen Initiatorsysteme, bezogen auf die Gesamtmenge der zu polymerisierenden Monomeren 0,1 bis 2 Gew.-%. Besonders bevorzugt werden Ammonium- und/oder Alkalimetallperoxidisulfate für sich oder als Bestandteil kombinierter Systeme als Initiatoren eingesetzt.

Die radikalische wäßrige Pfropfpolymerisation kann gegebenenfalls in Gegenwart von Reglern durchgeführt werden. Geeignete Regler sind beispielsweise Mercaptoverbindungen, wie Mercaptoethanol, Mercaptopropanol, Mercaptobutanol, Mercaptoessigsäure, Mercaptopropionsäure, Butylmercaptan und Dodecylmercaptan. Als Regler eignen sich außerdem Allylverbindungen wie Allylalkohol. Falls die Pfropfpolymerisation in Gegenwart von Reglern durchgeführt wird, kann man davon 0,05 bis 20 Gew.-%, bezogen auf die bei der Polymerisation eingesetzten Monomeren, verwenden.

Selbstverständlich kann die erfindungsgemäße radikalische wäßrige Polymerisation auch unter erhöhtem oder reduziertem Druck erfolgen.

Die wäßrigen Polymerisatdispersionen werden in der Regel mit Gesamtfeststoffgehalten von 15 bis 65, bevorzugt 30 bis 60 Gew.-% hergestellt.

Den Polymerisatdispersionen können übliche Zusatzstoffe zugesetzt werden. Sie werden in der Regel nach Polymerisationsende zugegeben. Genannt seien vernetzend wirkende nicht einpolymerisierbare Stoffe, die üblicherweise in Mengen von 0,1 bis 5 Gew.-% vorliegen können. Enthält das Polymerisat freie Carboxylgruppen, sind Verbindungen geeignet, die solche Gruppen vernetzen kannen wie basische Verbindungen mehrwertiger Metalle wie Zinkoxyd, Calciumoxyd oder die entsprechenden Hydroxyde, Acetate oder Carbonate oder die entsprechenden gemischten Salze. Weiterhin sind Verbindungen geeignet, die gegebenenfalls vorhandene Hydroxygruppen vernetzen, wie di- und polyfunktionelle anorganische Säuren und Säurederivate, z.B. Phosphoroxichlorid, Alkalitrimetaphosphate, Alkalipolyphosphate oder Alkalitetraborat, diund polyfunktionelle oganische Säuren, z.B. Adipinsäure, Zitronensäure, 1,2,3,4-Butantetracarbonsäure, all-cis-1,2,3,4-Cyclopentantetracarbonsäure, Derivate von di- und polyfunktionellen organischen Säuren, wie Anhydride oder gemischte Anhydride, z.B. Diacetyladipinsäure, Acetylzitronensäureanhydrid, Säurechloride, z.B. Cyanursäurechlorid, Imidazolide und Guanidinderivate, außerdem di- und polyfunktionelle Isocyanate wie Hexamethylendiisocyanat, 2,4-Diisocyanattoluol, di- und polyfunktionelle Alkylierungsmittel, z.B. Epichlorhydrin, β,β'-Dichlorethylether, Diepoxide, verschiedene Aldehyde oder Aldehydderivate wie Formaldehyd, Acetaldehyd, Acrolein, 2,5-Dimethoxytetrahydrofuran oder Glutardialdehyd. Ferner kommen Kondensationsprodukte auf der Basis Formaldehyd, Glyoxal, Melamin, Phenol und/oder Harnstoff in Betracht. Dabei sind Formaldehyd und die formaldehydhaltigen Vernetzersysteme aufgrund toxikologischer Bedenken weniger geeignet.

Die für gebundene Vliesstoffe üblichen Zusatzstoffe können in bekannten Mengen zugesetzt werden, wobei biologisch abbaubare Substanzen bevorzugt sind. Zum Erhalt der Produkteigenschaften sind die gebundenen Vliesstoffe oft frei von Ton oder Kreide.

Die Verfestigung der Faservliese mit den Polymerisaten erfolgt nach bekannten Methoden (z.B. Ullmann's Encyklopädie der technischen Chemie, 4. Auflage, Band 23, 1983, S. 738 bis 742). Üblicherweise wird das Faservlies durch Badimprägnieren, Schaumimprägnieren, Besprühen, Pflatschen oder Bedrucken mit der Polymerisatdispersion getränkt. Dazu kann die Dispersion evtl. mit Wasser verdünnt oder aber mit üblichen Verdickungsmitteln verdickt werden, um die gewünschte Verarbeitungsviskosität einzustellen. Der Vliesbehandlung mit der Dispersion schließt sich im allgemeinen eine Trocknung und Temperung des erhaltenen Vliesstoffes an. Die Trocknungsbedingungen hängen von der Art des eingesetzten Trockners ab, üblicherweise liegt die Trocknungstemperatur zwischen 100 und 230°C und die Trocknung bzw. Temperung wird zwischen 10 sek und 60 min durchgeführt.

Die erfindungsgemäßen Vliesstoffe zeichnen sich durch ihre gute Kompostierbarkeit bei günstigen Gebrauchseigenschaften aus. Sie weisen u.a. eine gute Trockenfestigkeit, eine hohe Naßfestigkeit und einen weichen Griff auf. Die hohe Naßfestigkeit der Produkte ist überraschend, da Saccharide hydrophile Stoffe sind, so daß eine hohe Naßfestigkeit im Grunde nicht zu erwarten war. Da Oligo- und Polysaccharide aufgrund der zahlreichen Wasserstoffbrückenbindungen als harte Stoffe bekannt sind, ist der weiche Charakter der erfindungsgemäßen Vliesstoffe ebenfalls überraschend.

### Beispiele

Als Polysaccharide wurden die Stärken C∗ PUR 01915 oder 01934 der Cerestar Deutschland GmbH, D-1150 Krefeld 12, eingesetzt. Sie weisen im wesentlichen alle eine bimodale Molekulargewichtsverteilung auf und sind wie folgt charakterisiert:

| Typ | M_{w} | U | Gew.-% < 1000 | DE | η⁴⁰ [Pa·s] |
|---|---|---|---|---|---|
| 01915 | 6680-8350 | 6,8-8,4 | 32,9-34,7 | 17-19 | 0,021 |
| 01934 | 3000 | 6,0 | 68,4 | 36-39 | 0,009 |

Eine Bestimmung von Mₙ mittels Dampfdruckosmose ergab für den Typ 01915 folgenden Wert:
980 g/mol

Die Bestimmung von M_{w} und U erfolgte durch Gelpermeationschromatographie nach üblichen Methoden, wobei folgende Parameter gewählt wurden:
- Säulen:: 3 Stück 7,5 x 600 mm Stahl gefüllt mit TSK-Gel G 2000 PW; G 3000 PW u. G 4000 PW. Porenw. 5 µm
- Eluent:: Wasser dest.
- Temp.:: RT (Raumtemperatur)
- Detektion:: Differentialrefraktometer (z.B. ERC 7511)
- Fluß:: 0,8 ml/min. Pumpe: (z.B. ERC 64.00)
- Injectv.:: 20 µl Ventil: (z.B. VICI 6-Wege-Ventil)
- Auswertung:: Bruker Chromstar GPC-Software
- Eichung:: Die Eichung erfolgte im niedermolekularen Bereich mit Glucose, Raffinose, Maltose und Maltopentose. Für den höhermolekularen Bereich wurden Pullulan-Standards mit einer Polydispersität < 1,2 verwendet.

### Beispiel 1

Ein Gemisch aus 766 g Wasser, 100 g Stärke 01915 (Fa. Cerestar) und 2,2 g eines handelsüblichen Emulgators auf Basis einer Alkyldiphenyloxidsulfonsäure (Dowfax@ 2A1 der Fa. Dow) wird auf 85°C erhitzt und mit 20 Gew.-% vom Zulauf 2 sowie 5 min später mit 10 Gew.-% vom Zulauf 1 versetzt. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, abgekühlt und bei einer Innentemperatur < 30°C 2,9 g t-Butylhydroperoxid (70 gew.-%ig in Wasser) zugesetzt. 5 min später gibt man eine Mischung aus 2 g Natriumsalz der Hydroxymethansulfinsäure, 0,1 g Armnoniumeisen-II-sulfat, 0,5 g Natriumsalz der Ethylendiamintetraessigsäure und 10 g Wasser über 15 min zu und rührt 30 min nach.

Feststoffgehalt der wäßrigen Polymerdispersion: ca. 44 Gew.-%, gemessen nach DIN 53 189.

Glastemperatur der verfilmten Polymerdispersion (Filmdicke: 0,5 mm, Trocknung: 30 h bei Raumtemperatur): -42°C
Zulauf 1:
   - 380 g: Wasser
   - 950 g: n-Butylacrylat
   - 20 g: Acrylsäure
   - 30 g: Acrylamidoglykolsäure
   - 2,2 g: Dowfax 2A1 (45 gew.-%ig in Wasser)
Zulauf 2:
   - 6 g: Natrimperoxidisulfat
   - 200 g: Wasser

### Beispiel 2

Ein Gemisch aus 365 g Wasser und 35 g Stärke 01915 wird auf 85°C erhitzt und mit 20 Gew.-% vom Zulauf 2 sowie 5 min später mit 10 Gew.-% vom Zulauf 1 versetzt. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, abgekühlt und bei einer Innentemperatur von 70°C 1,4 g t-Butylhydroperoxid (70 gew.-%ig in Wasser) zugesetzt. 5 min später gibt man eine Mischung aus 1 g Natriumsalz der Hydroxymethansulfinsäure, 0,05 g Ammoniumeisen-II-sulfat, 0,25 g Natriumsalz der Ethylendiamintetraessigsäure und 5 g Wasser über 60 min zu und kühlt auf Raumtemperatur ab.

Feststoffgehalt der wäßrigen Polymerdispersion: ca. 44 Gew.-%

Glastemperatur: -12°C (bestimmt gemäß den Angaben in Beispiel 1)
Zulauf 1:
   - 190 g: Wasser
   - 483 g: Ethylacrylat
   - 17,5 g: Acrylamidoglykolsäure
   - 5,6 g: Dowfax 2A1 (45 gew.-%ig in Wasser)
Zulauf 2:
   - 3 g: Natriumperoxidsulfat
   - 100 g: Wasser

### Beispiel 3

Ein Gemisch aus 882 g Wasser, 63 g Stärke 01915 und 2,8 g Dowfax® 2A1 wird auf 85°C erhitzt und mit 20 Gew.-% vom Zulauf 2 sowie 5 min später mit 10 Gew.-% vom Zulauf 1 versetzt. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, abgekühlt und bei 70°C 3,6 g t-Butylhydroperoxid (70 gew.-%ig in Wasser) zugesetzt. 5 min später gibt man eine Mischung aus 2,5 g Natriumsalz der Hydroxymethansulfinsäure, 0,13 g Ammoniumeisen-II-sulfat, 0,63 g Natriumsalz der Ethylendiamintetraessigsäure und 12,5 g Wasser über 15 min zu und rührt 30 min nach.

Festoffgehalt der wäßrigen Polymerdispersion: ca. 44 Gew.-%

Glastemperatur: -34°C (bestimmt gemäß den Angaben in Beispiel 1)
Zulauf 1:
   - 475 g: Wasser
   - 875 g: n-Butylacrylat
   - 300 g: Ethylacrylat
   - 25 g: Acrylsäure
   - 50 g: Acrylamidoglykolsäure
   - 14 g: Dowfax 2A1 (45 gew.-%ig in Wasser)
Zulauf 2:
   - 7,5 g: Natriumperoxidsulfat
   - 250 g: Wasser

### Beispiel 4

Ein Gemisch auf 383 g Wasser, 50 g Stärke 01934 und 1,11 g Dowfax 2A1 wird auf 85°C erhitzt und mit 20 Gew.-% vom Zulauf 2 sowie 5 min später mit 10 Gew.-% vom Zulauf 1 versetzt. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, abgekühlt und bei 70°C 1,4 g t-Butylhydroperoxid (70 gew.-%ig in Wasser) zugesetzt. 5 min später gibt man eine Mischung aus 1 g Natriumsalz der Hydroxymethansulfinsäure, 0,05 g Ammoniumeisen-II-sulfat, 0,25 g Natriumsalz der Ethylendiamintetraessigsäure und 5 g Wasser über 15 min zu und rührt 30 min nach.

Feststoffgehalt der wäßrigen Polymerdispersion: ca. 44 Gew.-%

Glastemperatur: -11°C (bestimmt nach den Angaben in Beispiel 1)
Zulauf 1:
   - 190 g: Wasser
   - 483 g: Ethylacrylat
   - 17,5 g: Acrylamidoglykolsäure
   - 5,6 g: Dowfax® 2A1 (45 gew.-%ig in Wasser)
Zulauf 2:
   - 3 g: Natriumperoxidsulfat
   - 100 g: Wasser

### Beispiel 5

Ein Gemisch aus 826 g Wasser, 2,5 g Natriumacetat und 62,5 g Stärke 01934 wird auf 85°C erhitzt, 20 % vom Zulauf 3 und 36 % vom Zulauf 1 zugegeben. Es wird 15 min bei 85°C anpolymerisiert und anschließend die Restmenge von Zulauf 1 sowie 27 % von Zulauf 3 über eine Stunde zudosiert. Nach Ende von Zulauf 1 werden Zulauf 2 und die Restmenge von Zulauf 3 über 1,5 h (Zulauf 2) bzw. 2 h (Zulauf 3) zugegeben. Danach wird 1 h bei 85°C nachpolymerisiert, auf 70°C abgekühlt und 8,3 g Wasserstoffperoxid (30 %ig in Wasser) zugesetzt. 5 min später gibt man eine Mischung aus 2,5 g Ascorbinsäure, 0,08 g Ammoniumeisen-II-sulfat und 13 g Wasser über 1 h zu, rührt 30 min nach und kühlt auf Raumtemperatur ab.

Gesamtfeststoffgehalt der wäßrigen Polymerdispersion: ca. 43 %

Glastemperatur: 0°C
Zulauf 1:
   - 140 g: Wasser
   - 338 g: Ethylacrylat
   - 12 g: Acrylamidoglykolsäure
   - 12 g: Steinapol NLS (15 %ig in Wasser)
Zulauf 2:
   - 360 g: Wasser
   - 287 g: Ethylacrylat
   - 581 g: Vinylpropionat
   - 32 g: Acrylamidoglykolsäure
   - 30 g: Steinapol NLS (15 %ig in Wasser)
   -
Zulauf 3:
   - 7,5 g: Natriumperoxidisulfat
   - 250 g: Wasser
   -

### [Steinapol NLS = Natriumlaurylsulfat]

### Beispiel 6

644 g Wasser werden vorgelegt, auf 85°C erhitzt und 20 % vom Zulauf 2 sowie 10 % vom Zulauf 1 zugegeben. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, auf 70°C abgekühlt und 2,9 g t-Butylhydroperoxid (70 %ig in Wasser) zugesetzt. 5 min später gibt man eine Mischung aus 2 g Natriumsalz der Hydroxymethansulfinsäure, 0,1 g Ammoniumeisen-II-sulfat, 0,5 g Natriumsalz der Ethylendiamintetraessigsäure und 10 g Wasser über 1 h zu, rührt 30 min nach und kühlt auf Raumtemperatur ab.

Gesamtfeststoffgehalt der wäßrigen Polymerdispersion: ca. 45 %

Glastemperatur: -11°C
Zulauf 1:
   - 380 g: Wasser
   - 965 g: Ethylacrylat
   - 35 g: Acrylamidoglykolsäure
   - 11,1 g: Dowfax 2 Al (45 %ig in Wasser)
   - 67 g: C₈-C₁₀-Glucosid (55 %ig in Wasser)
Zulauf 2:
   - 6 g: Natriumperoxidisulfat
   - 200 g: Wasser

Das eingesetzte C₈-C₁₀-Glucosid wurde gemäß der DE-A-4212080 nach der folgenden Vorschrift hergestellt.

In einem 2-1-Mehrhalsrührreaktor mit Strombecher, Scheibenrührer, Thermometer, Destillationsaufsatz und einer Dosiereinheit, bestehend aus einer Dosierpumpe, Druckhalteventil und einer Düse, wurden 663 g Lorol® C₈₋₁₀ Spezial (Henkel, Gemisch aus Octanol und Decanol) vorgelegt und dazu 2,6 g (0,008 mol) Dodecylbenzolsulfonsäure gegeben. Diese Mischung wurde mit 121,6 g eines Alkylglucosid/Alkohol-Emulgator-Gemenges (Zusammensetzung: 63 % Lorol, 22,6 % C₁₂-Monoglucosid, 5,2 % C₁₂-Diglucosid, 2,2 % C₁₂-Triglucosid, 0,7 % C₁₂-Tetraglucosid, < 0,5 % C₁₂-Pentaglucosid, 5,9 % Polyglucose) versetzt. Hierbei betrugen die Glucose-enthaltenden Anteile des Emulgators 30 % bezüglich der eingesetzten Glucose. Das molare Verhältnis von Fettalkohol zu Glucose betrug 6:1.

Die Lösung wurde auf 115 bis 120°C erhitzt. Bei einem Vakuum von 30 bis 35 mbar wurden kontinuierlich 214 g (0,83 mol) eines auf 60°C erwärmten Dextrosesirups (70 %ige Lösung, Glukose-Gehalt ca. 99,5 %) so zudosiert, daß eine trüb erscheinende Emulsion entsteht, die praktisch keine teilchenförmigen Ausfällungen an Polyglucose enthält. Nach 4 h Dosierzeit und 30 min Nachrührzeit waren 83 g Wasser abdestilliert. Man erhielt eine leicht trübe, hellgelbe Reaktionslösung.

Nach Abkühlen auf 90°C wurde mit 1,6 g 50 %iger Natronlauge der Katalysator desaktiviert, die resultierende Lösung hatte einen pH-Wert von 8,3 (gemessen in 50 %iger wäßriger Lösung). Der überschüssige Alkohol wurde mittels Dünnschichtverdampfer (Heiztemperatur 170°C, Ablauftemperatur 140°C) bei einem Vakuum von 1 mbar entfernt. Das Produkt wurde durch Wasserzugabe direkt zu einer wäßrigen Lösung verarbeitet und mit 12,3 g H₂0₂ (30 %ige Lösung) bei 80°C gebleicht.

### Beispiel 7

661 g Wasser werden vorgelegt, auf 85°C erhitzt und 20 % vom Zulauf 2 sowie 10 % vom Zulauf 1 zugegeben. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, auf 70°C abgekühlt und 2,9 g t-Butylhydroperoxid (70 %ig in Wasser) zugesetzt. 5 min später gibt man eine Mischung aus 2 g Natriumsalz der Hdroxymethansulfinsäure, 0,1 g Ammoniumeisen-II-sulfat, 0,5 g Natriumsalz der Ethylendiamintetraessigsäure und 10 g Wasser über 1 h zu, rührt 30 min nach und kühlt auf Raumtemperatur ab.

Gesamtfeststoffgehalt der wäßrigen Polymerdispersion: ca. 44 %

Glastemperatur: -11°C
Zulauf 1:
   - 380 g: Wasser
   - 965 g: Ethylacrylat
   - 35 g: Acrylamidoglykolsäure
   - 11,1 g: Dowfax 2 Al (45 %ig in Wasser)
   - 95 g: C₁₀-C₁₂-Glucosid (47 %ig in Wasser)
Zulauf 2:
   - 6 g: Natriumperoxidisulfat
   - 200 g: Wasser

Das eingesetzte C₁₀-C₁₂-Glucosid wurde wie in Beispiel 6, jedoch unter Verwendung von 754 g Nafol® 1012 (Fa. Condea, Gemisch aus Decanol, Dodecanol und geringe Mengen Tetradecanol) hergestellt.

### Vergleichsbeispiel 1

In einer 2 l fassenden Glasapparatur, die mit einem Ankerrührer, Zulaufeinrichtungen für Monomere, Initiatorlösungen und Ammoniaklösung, Rückflußkühler und Stickstoffein- und -auslaß ausgestattet ist, werden 120 g Kasein (in der Säureform vorliegend) in 500 g Wasser unter Stickstoff bei einer Temperatur von 20°C suspendiert. Man gibt dann in einer Portion 180 g n-Butylacrylat zu und rührt die Mischung 15 min bei 20°C. Danach werden 9 g 25 %ige wäßrige Ammoniaklösung innerhalb von 15 min tropfenweise zugegeben. Nach Beendigung der Ammoniakzugabe wird die Mischung noch 40 min bei 20°C gerührt. Man fügt dann 20 g einer 13 gew.-%igen Natriumperoxidsulfatlösung in einer Portion zu und erhöht die Temperatur des Reaktionsgemisches auf 75°C.

Sobald diese Temperatur erreicht ist, dosiert man 20 g einer 10 gew.-%igen Natriumperoxidsulfatlösung innerhalb von 2 h zu und rührt das Reaktionsgemisch noch 2 h bei 70°C nach Beendigung der Initiatorzugabe. Dann wird 1 g t-Butylperpivalat zugegeben und weitere 2 h bei 75°C gerührt.

### Vergleichsbeispiel 2

Beispiel 4 wurde wiederholt, mit dem Unterschied, daß die Stärke nach Polymerisationsende zugemischt wurde.

### Vergleichsbeispiel 3

Eingesetzt wurde ein Emulsionspolymerisat auf Basis Styrol/Butadien, hergestellt ohne Saccharid, mit einer Glastemperatur von -16°C.

### Vergleichsbeispiel 4

Ein Gemisch aus 832 g Wasser und 2,8 g Dowfax 2 Al wird auf 85°C erhitzt, 20 % vom Zulauf 2 sowie 10 % vom Zulauf 1 zugegeben. Es wird 15 min bei 85°C anpolymerisiert und anschließend mit der Dosierung der Restmengen der Zuläufe 1 und 2 begonnen. Die Zugabe erfolgt kontinuierlich über 2,5 h (Zulauf 1) und 3 h (Zulauf 2). Danach wird 1 h bei 85°C nachpolymerisiert, auf 70°C abgekühlt und 3,6 g t-Butylhydroperoxid (70 %ig in Wasser) zugesetzt. 5 min später gibt man eine Mischung aus 2,5 g Natriumsalz der Hydroxymethansulfinsäure, 0,13 g Ammoniumeisen-II-sulfat, 0,63 g Natriumsalz der Ethylendiamintetraessigsäure und 13 g Wasser über 1 h zu, rührt 30 min nach und kühlt auf Raumtemperatur ab.

Gesamtfeststoffgehalt der wäßrigen Polymerdispersion: ca. 44 %
Zulauf 1:
   - 475 g: Wasser
   - 875 g: Butylacrylat
   - 300 g: Ethylacrylat
   - 50 g: Hydroxyethylacrylat
   - 25 g: Acrylsäure
   - 14 g: Dowfax 2 Al (45 %ig in Wasser)
Zulauf 2:
   - 7,5 g: Natriumperoxidisulfat
   - 250 g: Wasser

Zu 700 Gew.-Teilen der Polymerdispersion wird eine Mischung aus 38,5 Gew.-Teilen einer Lösung von 1,3-Dimethyl-4,5-dihydroxiimidazolidin-2-on (40 %ig in Wasser) und 31 Gew.-Teilen Citronensäure (25 %ig in Wasser) gegeben und 5 min gerührt.

### Herstellung der Vliesstoffe:

Ein längsgelegtes (Faserorientierung bevorzugt in einer Richtung, der Längsrichtung) Faservlies aus Zellwollfasern mit einem Flächengewicht von 35 g/m² wurde in unabhängigen Versuchen mit den Dispersionen aus den angeführten Beispielen und Vergleichsbeispielen, die zuvor auf einen einheitlichen Feststoffgehalt von 10 % verdünnt worden waren, getränkt, zur Abtrennung des überschüssigen Dispersionsanteils zwischen zwei gegenläufige Walzen gebracht und danach 4 min einer Temperatur von 150°C ausgesetzt. Der Bindemittelanteil der so erhaltenen Vliesstoffe betrug in allen Fällen 25 Gew.-%.

### Untersuchungen an den gebundenen Vliesstoffen:

Aus den Vliesstoffen wurden 50 mm breite Streifen geschnitten und diese bei einer freien Einspannlänge von 10 cm im trockenen und im wassernassen Zustand zur Ermittlung der Reißfestigkeit in Analogie zur DIN 53 857 einem Streifenzugversuch ausgesetzt. Dabei wurden die Reißfestigkeiten (durch entsprechendes Ausschneiden der Prüfkörper) quer zur Faservorzugsrichtung gemessen.

Ferner wurden die Vliesstoffproben zur Ermittlung der Biegesteifigkeit, welche als Maß für die Weichheit herangezogen wird, um einen metallenen Dorn gebogen. Als Biegesteifigkeit wurde dabei die Kraft gemessen, die für das Biegen aufzuwenden war. Diese Kraft wurde quer zur Faservorzugsrichtung gemessen.

Die Ergebnisse dieser Versuche gehen aus Tabelle 1 hervor.

**Tabelle 1**

| Bespiele und Vergleichsbeispiele | Höchstzugkraft [N] | | Biegesteifigkeit [mN] |
|---|---|---|---|
| | trocken | wassernaß | |
| Beispiel 1 | 33 | 8 | 24 |
| Beispiel 2 | 34 | 9 | 41 |
| Beispiel 3 | 31 | 13 | 21 |
| Beispiel 4 | 33 | 8 | 24 |
| Beispiel 5 | | 13 | |
| Beispiel 6 | | 13 | |
| Beispiel 7 | | 11 | |
| Vergleichsbeispiel 1 | 91 | <1 | 130 |
| Vergleichsbeispiel 2 | | 12 | 20 |
| Vergleichsbeispiel 3 | 47 | 13 | 30 |
| Vergleichsbeispiel 4 | 10 | 6 | 20 |

Um die Kompostierbarkeit der erfindungsgemäß erhaltenen Vliesstoffe zu untersuchen, wurden diese in Anlehnung an den Erdeingrabtest nach DIN 53 933 untersucht, d.h. in Blumenerde eingegraben und darin über eine definierten Zeitraum belassen. Nach dem Ausgraben der Vliesstoffe wurde zum einen deren Reißfestigkeit bestimmt und daraus im Vergleich mit einer entsprechenden nicht eingegrabenen Probe der durch das Eingraben bedingte Verlust an Reißfestigkeit ermittelt. Dieser Verlust an Reißfestigkeit wird als Maß für die Kompostierbarkeit herangezogen. Daneben wurden die Vliesstoffproben visuell auf ihren Zerfallszustand hin begutachtet. Je höher der Reißfestigkeitsverlust und je stärker der visuell erkennbare Zerfall der Proben, desto besser kompostierbar können die Vliesstoffe gelten.

Die Vliesstoffe für die Kompostierversuche wurden in der oben beschriebenen Weise hergestellt, als Rohvlies wurde dabei jedoch ein leicht vorvernadeltes Zellwollvlies mit einem Flächengewicht von 50 g/m² verwendet. Die Ergebnisse der Kompostierversuche sind in Tabelle 2 zusammenfassend dargestellt:

**Tabelle 2**

| Beispiel | Reißfestigkeit (trocken) vor dem Eingraben | Reißfestigkeit (trocken) 9 d nach dem Eingraben |
|---|---|---|
| Beispiel 1 | 27 N | 5 N |
| Beispiel 3 | 25 N | 13 N |
| Vergleichsbeispiel 3 | 40 N | 30 N |
| Unverfestigtes Rohvlies | 10 N | 2 N |

Die visuelle Beurteilung der Vliesstoffproben ergab, daß die Proben nach dem Eingraben des Vliesstoffes mit Vergleichsbeispiel 3 gebunden kaum einen sichtbaren Zerfall aufwiesen. Die Vliesstoffe mit Beispiel 1 und Beispiel 3 gebunden zeigten hingegen deutliche Zerfallserscheinungen: die Proben waren spürbar dünner geworden und wiesen vereinzelt kleine Löcher auf.

## Patentansprüche

1. Gebundener Vliesstoff, enthaltend als Binder 5 bis 100 Gew.-%, bezogen auf das Gewicht der eingesetzten, biologisch abbaubaren Fasern, eines Polymerisates, das eine Glastemperatur von -70 bis +40°C aufweist und das herstellbar ist durch radikalische Polymerisation eines Gemisches ethylenisch ungesättigter Monomerer in wäßrigem Medium in Gegenwart eines Saccharids mit einem gewichtsmittleren Molekulargewicht von 1000 bis 25000, wobei das Monomerengemisch 0,5 bis 15 Gew.-% N-Alkylolamide von 3 bis 10 C-Atome aufweisenden α,β-monoethylenisch ungesättigten Carbonsäuren, Acrylamidoglykolsäure, Methacrylamidoglykolsäure und/oder deren Ether, Ester oder Etherester mit Alkoholen mit bis zu 12 C-Atomen, enthält.

2. Gebundener Vliesstoff nach Anspruch 1, wobei als Saccharid Dextrin eingesetzt wird.

3. Gebundener Vliesstoff nach Anspruch 1, wobei als Saccharid ein durch Hydrolyse in wäßriger Phase erhältliches Abbauprodukt von Stärke eingesetzt wird.

4. Gebundener Vliesstoff nach Anspruch 1, wobei als Saccharid ein Acetal eines Mono- oder Oligosaccharides mit C₆- bis C₁₈-Alkanolen eingesetzt wird.

5. Gebundener Vliesstoff nach einem der Ansprüche 1 bis 4, wobei das Polymerisat aus
- 70 bis 99,5 Gew.-% aus Estern der Acryl- und/oder Methacrylsäure mit 1 bis 12 C-Atome aufweisenden Alkanolen, Styrol, Butadien, Vinylacetat und/oder Vinylpropionat,
- 0,5 bis 15 Gew.-% N-Alkylolamide von 3 bis 10 C-Atome aufweisenden α,β-monoethylenisch ungesättigten Carbonsäuren, Acrylamidoglykolsäure, Methacrylamidoglykolsäure und/oder deren Ether, Ester oder Etherester mit Alkoholen mit bis zu 12 C-Atomen,
- 0 bis 15 Gew.-% weiterer Monomerer
aufgebaut ist.

6. Verfahren zum Verfestigen von Faservliesen, dadurch gekennzeichnet, daß man 5 bis 100 Gew.-%, bezogen auf das Gewicht der eingesetzten, biologisch abbaubaren Fasern, eines Polymerisates, das eine Glastemperatur von -70 bis +40°C aufweist und das herstellbar ist durch radikalische Polymerisation eines Gemisches ethylenisch ungesättigter Monomerer in wäßrigem Medium in Gegenwart eines Saccharids mit einem gewichtsmittleren Molekulargewicht von 1000 bis 25000, einsetzt, wobei das Monomerengemisch 0,5 bis 15 Gew.-% N-Alkylolamide von 3 bis 10 C-Atome aufweisenden α,β-monoethylenisch ungesättigten Carbonsäuren, Acrylamidoglykolsäure, Methacrylamidoglykolsäure und/oder deren Ether, Ester oder Etherester mit Alkoholen mit bis zu 12 C-Atomen, enthält.

7. Verwendung des gebundenen Vliesstoffes nach einem der Ansprüche 1 bis 6 zur Herstellung von Windeln.

## Claims

1. A nonwoven bonded with from 5 to 100% by weight, based on the weight of the biodegradable fibers used, of a polymer which has a glass transition temperature of from -70 to +40°C and is preparable by free radical polymerization of a mixture of ethylenically unsaturated monomers in an aqueous medium in the presence of a saccharide having a weight average molecular weight of from 1000 to 25000, the monomer mixture comprising from 0.5 to 15% by weight of N-alkylolamides of α,β-monoethylenically unsaturated carboxylic acids having from 3 to 10 carbon atoms, acrylamidoglycolic acid, methacrylamidoglycolic acid or their ethers, esters or ether-esters with alcohols having up to 12 carbon atoms.

2. The nonwoven of claim 1, wherein the saccharide used is dextrin.

3. The nonwoven of claim 1, wherein the saccharide used is a starch degradation product obtainable by aqueous phase hydrolysis.

4. The nonwoven of claim 1, wherein the saccharide used is an acetal of a mono- or oligosaccharide with alkanols having from 6 to 18 carbon atoms.

5. The nonwoven of claim 1, wherein the polymer is formed from
- from 70 to 99.5% by weight of esters of acrylic and/or methacrylic acid with alkanols having from 1 to 12 carbon atoms, styrene, butadiene, vinyl acetate and/or propionate,
- from 0.5 to 15% by weight of N-alkylolamides of α,β-monoethylenically unsaturated carboxylic acids having from 3 to 10 carbon atoms, acrylamidoglycolic acid, methacrylamidoglycolic acid and/or their ethers, esters or ether-esters with alcohols having up to 12 carbon atoms,
- from 0 to 15% by weight of other monomers.

6. A process for consolidating fiber webs, which comprises using from 5 to 100% by weight, based on the weight of the biodegradable fibers used, of a polymer which has a glass transition temperature of from -70 to +40°C and is preparable by free radical polymerization of a mixture of ethylenically unsaturated monomers in an aqueous medium in the presence of a saccharide having a weight average molecular weight of from 1000 to 25000, the monomer mixture comprising from 0.5 to 15% by weight of N-alkylolamides of α,β-monoethylenically unsaturated carboxylic acids having from 3 to 10 carbon atoms, acrylamidoglycolic acid, methacrylamidoglycolic acid or their ethers, esters or ether-esters with alcohols having up to 12 carbon atoms.

7. The use of the nonwoven of any of claims 1 to 6 for manufacturing diapers.

## Revendications

1. Non-tissé lié, contenant comme liant 5 à 100% en poids, par rapport au poids des fibres utilisées dégradables biologiquement, d'un polymère, qui présente une température de transition vitreuse de -70°C + 40°C et qui est fabriquable par polymérisation radicalaire d'un mélange de monomères à insaturation éthylénique en milieu aqueux en présence d'un saccharide de poids moléculaire moyen de 1000 à 25000, dans lequel le mélange de monomères contient 0,5 à 15% en poids de N-alkylolamides d'acides carboxyliques à insaturation α, β-monoéthylénique ayant 3 à 10 atomes de carbone, d'acide acrylamidoglycolique, d'acide méthacrylamidoglycolique et/ou leurs éthers, esters ou éther-esters avec des alcools ayant jusqu'à 12 atomes de carbone.

2. Non-tissé lié selon la revendication 1, dans lequel on utilise la dextrine en tant que saccharide.

3. Non-tissé lié selon la revendication 1, dans lequel on utilise, en tant que saccharide, un produit de dégradation de l'amidon, obtenu par hydrolyse en phase aqueuse.

4. Non-tissé lié selon la revendication 1, dans lequel on utilise, en tant que saccharide, un acétal d'un mono- ou oligosaccharide avec des alcanols en C₆ à C₁₈.

5. Non-tissé lié selon l'une des revendications 1 à 4, dans lequel le polymère est élaboré à partir de :
• 70 à 99,5% en poids d'esters d'acide acrylique et/ou méthacrylique avec des alcanols ayant 1 à 12 atomes de carbone, du styrène, du butadiène, de l'acétate de vinyle et/ou du propionate de vinyle,
• 0,5 à 15% en poids de N-alkylolamides d'acides carboxyliques à insaturation α, β- monoéthylénique ayant 3 à 10 atomes de carbone, d'acide acrylamidoglycolique, d'acide méthacrylamidoglycolique et/ou leurs éthers, esters ou éther-esters avec des alcools ayant jusqu'à 12 atomes de carbone.
• 0 à 15% en poids d'autres monomères.

6. Procédé de solidification de non-tissés de fibres, caractérisé en ce qu'on utilise 5 à 100% en poids, par rapport au poids des fibres utilisées dégradables biologiquement, d'un polymère, qui présente une température de transition vitreuse de -70°C à + 40°C et qui est fabriquable par polymérisation radicalaire d'un mélange de monomères à insaturation éthylénique en milieu aqueux en présence d'un saccharide de poids moléculaire moyen de 1000 à 25000, dans lequel le mélange de monomères contient 0,5 à 15% en poids de N-alkylolamides d'acides carboxyliques à insaturation α, β- monoéthylénique ayant 3 à 10 atomes de carbone, d'acide acrylamidoglycolique, d'acide méthacrylamidoglycolique et/ou leurs éthers, esters ou éther-esters avec des alcools ayant jusqu'à 12 atomes de carbone.

7. Utilisation du non-tissé lié selon l'une des revendications 1 à 6 pour la fabrication de couches.
